# EUROPÄISCHE PATENTSCHRIFT

(11) **EP 3 810 220 B1**
(45) Veröffentlichungstag und Bekanntmachung des Hinweises auf die Patenterteilung: **19.02.2025**
(21) Anmeldenummer: 19730761.4
(22) Anmeldetag: 13.06.2019
(51) Int. Cl.: A61M 1/36, A61B 17/132, A61B 17/00

(54) **IMPLANTAT ZUR BEREITSTELLUNG EINES SHUNTS MIT VERSTELLBAREM DURCHFLUSS**
IMPLANT FOR THE REALISATION OF A SHUNT WITH ADJUSTABLE FLOW
IMPLANT POUR LA RÉALISATION D'UN SHUNT À DÉBIT RÉGLABLE

(30) Priorität: 20.06.2018 DE 102018114856
(43) Veröffentlichungstag der Anmeldung: 28.04.2021
(73) Patentinhaber: Fresenius Medical Care Deutschland GmbH, 61352 Bad Homburg (DE)
(72) Erfinder: RUDOLPH, Stefan, 99634 WERNINGSHAUSEN (DE)
(74) Vertreter: Herrmann, Uwe
(86) Internationale Anmeldenummer: PCT/EP2019/065508
(87) Internationale Veröffentlichungsnummer: WO 2019/243155

(56) Entgegenhaltungen:
- WO-A1-2012/104848
- US-A1- 2006 129 083
- US-A1- 2010 030 322
- US-A1- 2011 270 288

## Beschreibung

Die Erfindung betrifft ein Implantat zur Bereitstellung eines Shunts mit verstellbarem Durchfluss sowie ein System umfassend ein solches Implantat und ein passendes Betätigungselement.

Um für eine erfolgreiche Dialysebehandlung eine ausreichende Menge an Blut in der Vene des Patienten zur Verfügung zu haben, wird in der Regel die zu punktierende Vene mit einer daneben befindlichen Arterie operativ über einen Shunt verbunden (meist am Unterarm zwischen der *A. radialis* und der *V. cephalica*). Diese Verbindung ist dann permanent, d.h., auch zwischen Dialysebehandlungen offen und es fließt ständig eine große Menge Blut mit hohem Druck in die Vene. Dies kann potentiell zu Beeinträchtigungen beim Patienten führen, beispielsweise zu kardialen Problemen aufgrund des erhöhten Rückflusses über die Vene oder zu Durchblutungsstörungen in den Gliedmaßen nach der Verbindungsstelle, da über den Shunt zu viel Blut aus der Arterie direkt in die Vene übertritt.

Im Stand der Technik sind Implantate zur Bereitstellung gattungsgemäßer Shunts vorgeschlagen, welche die Verstellung des Durchflusses erlauben.

So offenbart die US 8,057,421 B2 einen Shunt, wobei der Blutfluss durch diesen Shunt mittels zweier Drehventile im Zu- und Ablauf des Shunts dosiert werden kann. Dabei wird je ein federbelastetes Drehventil mit Hilfe einer Hydrauliklösung gegen die Federkraft verstellt und damit der Durchfluss durch den Shunt verändert. Die Hydraulikflüssigkeit wird mittels einer Spritze über eine Zugangsöffnung zugegeben und abgezogen.

In der US 8,652,084 B2 wird eine Weiterentwicklung dieses Systems offenbart, wobei eines der Drehventile durch einen Schieber mit einer Öffnung und mit verschiedenen Sensoren ersetzt ist. Der Schieber kann ebenfalls hydraulisch betätigt werden und kann den Fluss durch den Shunt drosseln oder auch komplett unterbrechen. Die Sensoren liegen auf der dem Blutgefäß zugewandten Seite des Schiebers und versorgen eine Steuerelektronik zur Regelung der Schieberposition.

Die Offenlegungsschrift US 2010/0056978 A1 offenbart mehrere Ausführungsformen eines Shunts mit Mitteln zur Verengung des Shunts und Verringerung des Durchflusses. In einer Variante ist ein Gummiring vorgesehen, der von der Hautoberfläche mit dem Finger innerhalb des Ventils verschoben und in eine definierte Position gebracht werden kann, in welcher er den Shunt verengt. Wird der Ring zurückgeschoben, kann mehr Blut durch den Shunt fließen. In einer zweiten Variante besteht das Ventil aus einem festen Ventilkörper und es wird von außen mittels einer Spritze eine Flüssigkeit zwischen Ventilkörper und Shunt gebracht. So wird der Shunt verengt und der Durchfluss reduziert. Um den Durchfluss zu erhöhen, muss die Flüssigkeit wieder abgesaugt werden. In einer weiteren Variante umfasst das Ventil einen Käfig aus einem speziellen Metall sowie zwei damit verbundene Metallringe. Mit Hilfe eines elektromagnetischen Feldes wird in den Metallringen ein Strom induziert, der den Käfig erwärmt, wodurch der Ventilkörper seine Form verändert, was Auswirkungen auf den Durchfluss hat.

Die WO 2012/104848A1 offenbart ein Implantat mit einem kollabierbaren Stößel. Aus der US 2010/030322A1 ist ein Implantat mit einem selbst-expandieren Shunt bekannt, welcher mittels einer Manschette verengbar ist. Zudem offenbart die US2006/129083A1 Implantate, bei denen der Strömungsquerschnitt mittels aufblasbarer Blasen oder beweglicher Membranen veränderbar ist.

Da bei Dialysepatienten ein erhöhter Blutfluss zur Vene praktisch nur für die Zeit der Dialyse, also beispielsweise drei Mal pro Woche für etwa fünf Stunden notwendig ist, wäre es wünschenswert, den Shunt nur während dieser Zeit zu öffnen oder zu erweitern. In der restlichen Zeit könnte die Verbindung zumindest teilverschlossen werden, um die negativen Auswirkungen zu reduzieren. Die oben vorgestellten Lösungen aus dem Stand der Technik sind hierfür aber nur begrenzt geeignet. Eine Anpassung einer Flüssigkeitsmenge mittels Spritze vor und nach jeder Dialysebehandlung wäre ebenso wenig praktikabel wie die Verwendung eines Ventils mit einer komplizierten Sensorik oder filigranen Mechanik.

Aufgabe der Erfindung ist es, ein Konzept zur Verfügung zu stellen, mit dem ein Verschluss oder Teilverschluss eines Shunts zwischen Dialysebehandlungen in verbesserter Weise realisiert werden kann.

Vor diesem Hintergrund betrifft die Erfindung ein Implantat zur Bereitstellung eines Shunts mit verstellbarem Durchfluss, wobei das Implantat ein Ventilelement mit einem Durchlass umfasst, wobei das Ventilelement einen zwischen einer Durchlassposition und einer Schließposition verfahrbaren Stößel umfasst, der einen Permanentmagneten aufweist und derart federbelastet ist, dass er in seine Schließposition vorgespannt ist. Das Implantat kann so ausgebildet sein, dass der Durchfluss regel- oder steuerbar ist.

Die Schließposition entspricht also der Ruheposition des Stößels, die er aufgrund der Federbelastung einnimmt, sofern keine gegenläufige Krafteinwirkung erfolgt. Befindet sich der Stößel in seiner Schließposition, so wird der Querschnitt des Durchlasses verringert und so ein Durchfluss durch den Durchlass bzw. eine darin befindliche Leitung oder ein darin befindliches Blutgefäß behindert oder verhindert. Das Ventilelement ist also so ausgebildet, dass es in seiner Grundstellung geschlossen ist. Während einer Dialysebehandlung kann durch Anlegen eines externen Magnetfeldes und dessen Wechselwirkung mit dem Permanentmagneten des Stößels das Ventilelement temporär geöffnet werden. Nach Ende der Dialysebehandlung kann das externe Magnetfeld entfernt werden, sodass sich das Ventilelement aufgrund der Federbelastung des Stößels wieder von selbst schließt. Dieses Prinzip ist sehr einfach und nicht fehleranfällig. Ein Eingriff in den Körper des Patienten zur Manipulation des Ventils ist nicht erforderlich. Es ist ausreichend, ein externes Magnetfeld anzulegen, beispielsweise indem vor Beginn der Dialysebehandlung eine magnetische Manschette an den Arm des Patienten gelegt wird. Der Stößel kann eine oder mehrere Permanentmagneten umfassen.

In einer Ausführungsform ist vorgesehen, dass das Implantat eine durch den Durchlass verlaufende Leitung mit zumindest abschnittsweise flexiblen Wandungen umfasst. Geeignete Leitungen umfassen flexible Schlauchleitungen aus Kunststoffen wie beispielsweise Polyethylenterephthalat (PET, Markenname Dacron), Polytetrafluorethylen (PTFE, Markenname Gore-Tex) oder Silikon. Die Leitung steht vorzugsweise beidseitig mit einer gewissen Länge über den Durchlass des Ventilelements über, sodass eine einfachere Verbindung mit Blutgefäßen des Patienten ermöglicht wird.

In einer Ausführungsform ist vorgesehen, dass der Stößel anhand einer Druckfeder wie beispielsweise einer Schraubendruckfeder, einer Tellerfeder oder einer Gummifeder federbelastet ist. Derartige mechanische Federn sind einfach und wenig fehleranfällig. Sie sind in stark miniaturisierter Form verfügbar und haben einen geringen Platzbedarf. Es können eine oder mehrere derartige Federn auf den Stößel wirken.

In einer Ausführungsform ist vorgesehen, dass der Stößel in seiner Schließposition in den Durchlass ragt. Der Stößel sorgt also unmittelbar für eine Querschnittsveränderung des Durchlasses und drückt im Falle des Vorhandenseins einer durch den Durchlass verlaufenden Leitung unmittelbar auf die Leitung. In seiner Durchlassposition kann der Stößel entweder gar nicht oder nur in kleinerem Ausmaß in den Durchlass ragen. Maßgeblich ist, dass der in seiner Schließposition weiter in den Durchlass ragt als in seiner Durchlassposition und somit der Querschnitt des Durchlasses in der Schließposition des Stößels durch dessen Körper verkleinert wird.

In einer Ausführungsform ist vorgesehen, dass das Ventilelement ein Hydrauliksystem mit einem Angriffsbereich und einem am Durchlass angeordneten Wirkungsbereich aufweist, wobei der Stößel in seiner Schließposition derart Druck auf den Angriffsbereich ausübt, dass Hydraulikflüssigkeit verdrängt wird und am Wirkungsbereich des Hydrauliksystems eine Querschnittsverkleinerung des Durchlasses bewirkt. In dieser Variante sorgt der Stößel also mittelbar für eine Querschnittsveränderung des Durchlasses, indem er auf ein Hydrauliksystem wirkt. Bei der Hydraulikflüssigkeit kann es sich beispielsweise um Kochsalz handeln. Auch eine derartige mittelbare Aktivierung ist sehr einfach, miniaturisierbar und fehlerunanfällig.

In einer Ausführungsform ist vorgesehen, dass das Hydrauliksystem im Wirkungsbereich eine Ausstülpung aufweist, welche in den Durchlass ragt, wenn der Stößel in seiner Schließposition derart Druck auf den Angriffsbereich ausübt. Das Hydrauliksystem kann also über eine Öffnung direkt in den Durchlass münden, wobei die Hydraulikflüssigkeit anhand einer vorzugsweise flexiblen Membran vom Durchlass getrennt bleibt. Bei Druckausübung des Stößels auf den Angriffsbereich des Hydrauliksystems wird die Membran durch die verdrängte Flüssigkeit ausgeformt und ragt in den Durchlass. Mithilfe einer solchen Ausstülpung kann eine sehr sanfte Verengung des Durchlasses bzw. der Leitung erreicht werden, sodass Blutbestandteile nicht mechanisch beschädigt werden. Anhand einer solchen Ausstülpung kann auch eine mehrseitige Verengung des Durchlasses erreicht werden, indem sich die von der Membran verschlossene Öffnung des Hydrauliksystems über einen bestimmten Mindestwinkelbereich des Durchlassumfangs erstreckt, beispielsweise über mindestens einen Viertelkreis oder mindestens einen Halbkreis.

In einer Ausführungsform ist vorgesehen, dass das Ventilelement eine am Durchlass angeordnete Schließmechanik aufweist und der Stößel derart mit dieser Schließmechanik in Verbindung steht, dass der Querschnitt des Durchlasses in der Schließposition des Stößels verkleinert wird. In dieser Variante sorgt der Stößel mittelbar für eine Querschnittsveränderung des Durchlasses, indem er auf eine geeignete Schließmechanik wirkt. Anhand einer Schließmechanik kann beispielsweise eine mehrseitige Verengung des Querschnitts erreicht werden.

In einer Ausführungsform ist vorgesehen, dass die Schließmechanik eine Irisblende mit mehreren Lamellen umfasst, die über eine Mechanik gemeinsam in den Durchlass geschwenkt werden können. Derartige Blenden sind aus Fotokameras bekannt. Anhand einer derartigen Mechanik lässt sich der Querschnitt des Durchlasses bei feststehendem Mittelpunkt und fortwährender Kreisförmigkeit der Öffnung. Der Stößel kann dabei die Verdrehung bewirken.

In einer Ausführungsform kann auch vorgesehen sein, dass der Stößel direkt oder indirekt auf eine im Anwendungsfall blutdurchströmte Steuerbohrung des Ventilelements wirkt, um deren Querschnitt zu verändern. In dieser Weise kann der arterielle Blutdruck unterstützend bei der Betätigung des Ventils, d.h., bei der Querschnittsanpassung des Durchlasses wirken.

In einer Ausführungsform ist vorgesehen, dass es sich bei dem Ventilelement um ein Servoventil handelt, welches eine im Durchlass angeordnete Ventilkammer umfasst, die anhand einer Durchlassmembran sowohl von einem den Vorlauf bildenden Abschnitt des Durchlasses als auch von einem den Rücklauf bildenden Abschnitt des Durchlasses getrennt ist, wobei die Durchlassmembran lösbar auf dem den Rücklauf bildenden Durchlassabschnitt aufliegt und zwei Rücklauföffnungen aufweist, welche die Ventilkammer mit diesem Durchlassabschnitt verbinden, wobei eine der Rücklauföffnungen durch ein mit dem Stößel in Verbindung stehendes Schließelement lösbar verschlossen ist, und wobei die Durchlassmembran ferner eine Vorlauföffnung aufweist, welche die Ventilkammer mit dem den Vorlauf bildenden Durchlassabschnitt verbindet. Die Vorlauföffnung hat vorzugsweise in etwa dieselbe Querschnittsfläche wie die nicht durch das Schließelement verschlossene Rücklauföffnung.

Weiterhin betrifft die Erfindung ein System umfassend ein erfindungsgemäßes Implantat sowie ein magnetisches Betätigungselement. Das Betätigungselement dient der Aktivierung des Stößelmagneten, sodass eine Gegenkraft zu der Federspannung aufgebaut und das Ventil geöffnet werden kann.

In einer Ausführungsform ist vorgesehen, dass es sich bei dem magnetischen Betätigungselement um eine Armmanschette mit einem Gegenmagneten handelt. Die Manschette ist geeignet, an den Unterarm eines Patienten angelegt zu werden. Beispielsweise kann es sich um eine Klettmanschette aus einem flexiblen Textilmaterial handeln, wie sie aus Blutdruckmessgeräten bekannt ist. Der Gegenmagnet kann in die Manschette eingearbeitet sein. Es kann sich beispielsweise um einen Permanentmagneten handeln.

Anhand des erfindungsgemäßen Implantats und Systems kann ein Verfahren zur Behandlung eines Dialysepatienten realisiert werden, wobei vor Beginn der Dialysebehandlung ein externes Magnetfeld an den Arm des Patienten gelegt wird, um das Ventilelement zu öffnen und so einen hohen Blutfluss durch den Shunt zu ermöglichen. Nach Beendigung des Dialyseverfahrens kann das Magnetfeld entfernt werden, sodass sich das Ventilelement selbst schließt und der Blutfluss durch den Shunt bis zur nächsten Behandlungseinheit wieder gedrosselt oder unterbunden wird.

Das erfindungsgemäße Implantat kann einem Patienten im Rahmen einer Operation eingesetzt werden. Dabei ist denkbar, dass das Implantat in eine arteriovenöse Fistel eingesetzt wird oder so an eine arteriovenöse Fistel angelegt wird, dass die Fistel durch den Durchlass verläuft. Sofern das Implantat eine Leitung umfasst, kann diese Leitung an ihren beiden Enden mit den natürlichen Blutgefäßen des Patienten verbunden werden und zumindest einen Teil der Fistel ausbilden. Die arteriovenöse Fistel wird dabei in der Regel im Rahmen derselben Operation geschaffen. Denkbar ist aber auch ein nachträglicher Einsatz des Implantats. Vorzugsweise besteht die Fistel im Unterarm zwischen der *A. radialis* und der V. *cephalica.*

Weitere Einzelheiten und Vorteile der Erfindung ergeben sich aus den nachfolgend anhand der Figuren dargestellten Ausführungsbeispielen. In den Figuren zeigen:
- Figur 1:: eine schematische Darstellung einer ersten Ausführungsform eines erfindungsgemäßen Implantats;
- Figur 2:: eine schematische Darstellung einer zweiten Ausführungsform eines erfindungsgemäßen Implantats;
- Figur 3:: eine schematische Darstellung einer weiteren möglichen Ausgestaltung eines Ventils eines erfindungsgemäßen Implantats; und
- Figur 4:: eine schematische Darstellung einer wiederum anderen möglichen Ausgestaltung eines Ventils eines erfindungsgemäßen Implantats.

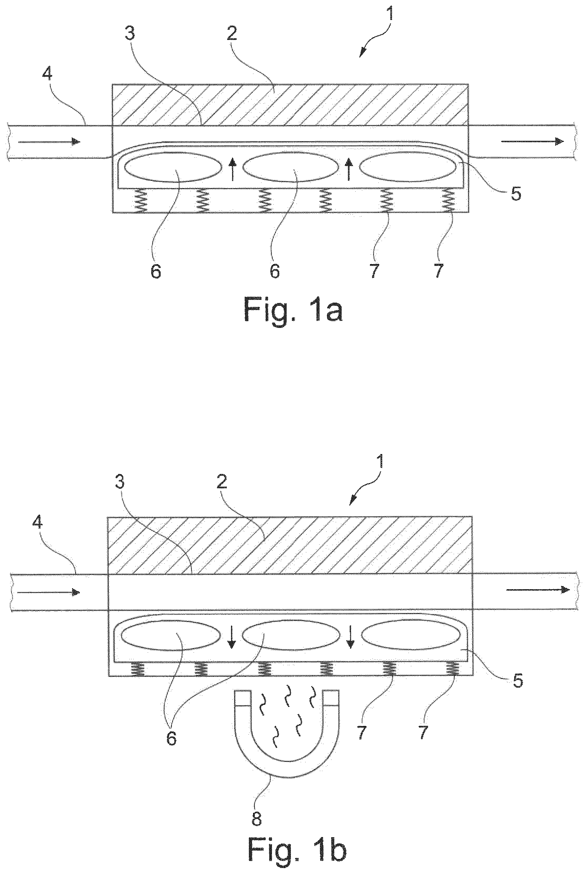

In Figur 1 wird eine erste Ausführungsform eines erfindungsgemäßen Implantats in zwei Zuständen gezeigt, nämlich mit verengtem Durchlass im inaktiven Zustand (Figur 1a) und mit erweitertem Durchlass im aktiven Zustand (Figur 1b).

Das Implantat 1 umfasst einen Ventilkörper 2 und einen Durchlass 3, durch den eine flexible Leitung 4 aus einem gewobenen PTFE-Material geführt ist. Die Leitung 4 stellt im Körper des Patienten eine arteriovenöse Fistel im Unterarm zwischen der *A. radialis* und der V. *cephalica* dar.

Zur Regulierung des Durchmessers des Durchlasses 3 und mithin des Strömungsquerschnitts der darin aufgenommenen Leitung 4 ist im Ventilkörper 2 ein Stößel 5 aufgenommen, der translatorisch zwischen einer Schließposition (Figur 1a) und einer Durchlassposition (Figur 1b) verfahren werden kann. Der Stößel 5 umfasst mehrere gleichmäßig über seine Fläche verteilte Permanentmagneten 6 und ist anhand mehrerer über seine Fläche verteilt angeordneter Schraubendruckfedern 7 gegen die Schließposition (Figur 1a) vorgespannt.

Der Stößel 5 ragt in seiner Schließposition (Figur 1a) direkt in den Durchlass 3 und übt Druck auf die Leitung 4 aus, sodass deren Strömungsquerschnitt verengt wird. So wird der Shunt zumindest teilweise verschlossen. In seiner Durchlassposition (Figur 1b) verringert der Stößel 5 den Strömungsquerschnitt der Leitung 4 nicht. Er ragt nicht in den Durchlass 3.

Wie dies in Figur 1b schematisch dargestellt ist, kann der Stößel 5 durch Annäherung eines externen Gegenmagneten 8 von der Schließposition (Figur 1a) in die Durchlassposition (Figur 1b) gezogen werden, da die magnetische Anziehungskraft zwischen den Permanentmagneten 6 des Stößels 5 und dem externen Gegenmagneten 8 die entgegengesetzte Federkraft der Schraubendruckfedern 7 übersteigt. Wird der Gegenmagnet 8 entfernt, bewegt sich der Stößel 5 aufgrund der Rückstellkraft der Schraubendruckfedern 7 wieder in seine Ausgangsposition zurück.

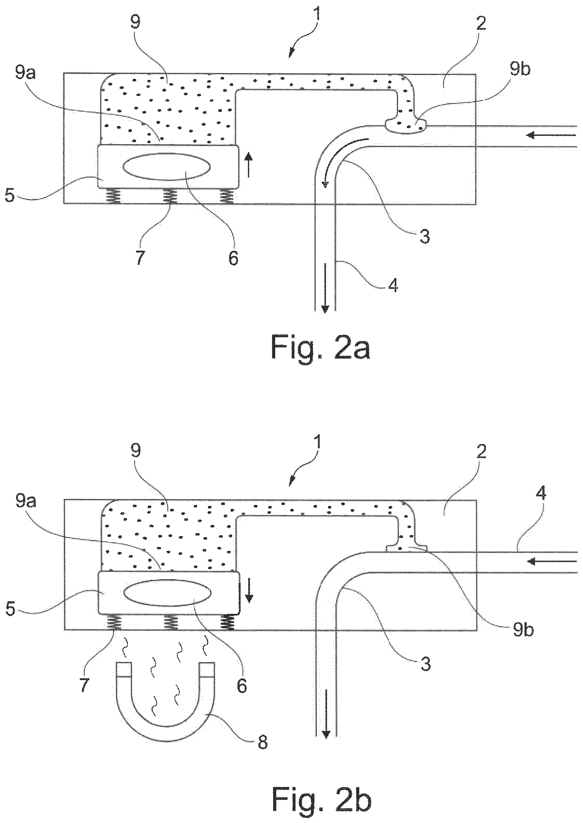

In Figur 2 wird eine zweite Ausführungsform eines erfindungsgemäßen Implantats in zwei Zuständen gezeigt, nämlich mit verengtem Durchlass im inaktiven Zustand (Figur 2a) und mit erweitertem Durchlass im aktiven Zustand (Figur 2b). Korrespondierende Elemente sind mit identischen Bezugszeichen versehen wie in Figur 1.

Im Gegensatz zur ersten Ausführungsform ragt der Stößel 5 in der zweiten Ausführungsform auch in seiner Schließstellung (Figur 2a) nicht in den Durchlass 3. Stattdessen ist ein Hydrauliksystem 9 im Ventilkörper 2 vorgesehen, das ein mit dem Stößel in Verbindung stehendes Reservoir 9a und einen am Durchlass 3 liegenden Wirkungsbereich 9b umfasst. Das Hydrauliksystem 9 ist mit einer geeigneten Lösung, die vorzugsweise biokompatibel ist, wie beispielsweise Kochsalzlösung, gefüllt.

In seiner Schließstellung (Figur 2a) übt der Stößel 5 Druck auf das Reservoir 9a des Hydrauliksystems 9 aus, sodass Hydraulikflüssigkeit aus dem Reservoir 9a verdrängt wird. Dies führt zu einer Ausstülpung einer Membran am Wirkungsbereich 9b des Hydrauliksystems 9 in den Durchlass 3. Diese Ausstülpung übt Druck auf die Leitung 4 aus, sodass deren Strömungsquerschnitt verengt wird. So wird der Shunt zumindest teilweise verschlossen.

In seiner Durchlassposition (Figur 2b) übt der Stößel 5 keinen Druck auf das Reservoir 9a des Hydrauliksystems 9 aus. Die Ausstülpung am Wirkungsbereich 9b des Hydrauliksystems 9 bildet sich zurück und ragt nicht mehr in den Durchlass 3.

Der Aufbau und die Mechanik des Stößels 5 sind im Wesentlichen identisch zur ersten Ausführungsform.

Bei Dialysepatienten ist ein erhöhter Blutfluss von der *A. radialis* in die *V. cephalica* nur für die Zeit der Dialyse notwendig. Zwischen den Behandlungen ist er nicht notwendig und kann gegebenenfalls sogar zu Komplikationen führen. Anhand der vorgestellten Implantate 1 kann also realisiert werden, dass der Strömungsquerschnitt der als Shunt dienenden Leitung 4 nur während einer Dialysebehandlung durch Annäherung eines externen Magneten 8, beispielsweise durch Anlegen einer magnetischen Klettmanschette erweitert wird, während er zwischen Dialysebehandlungen verengt bleibt.

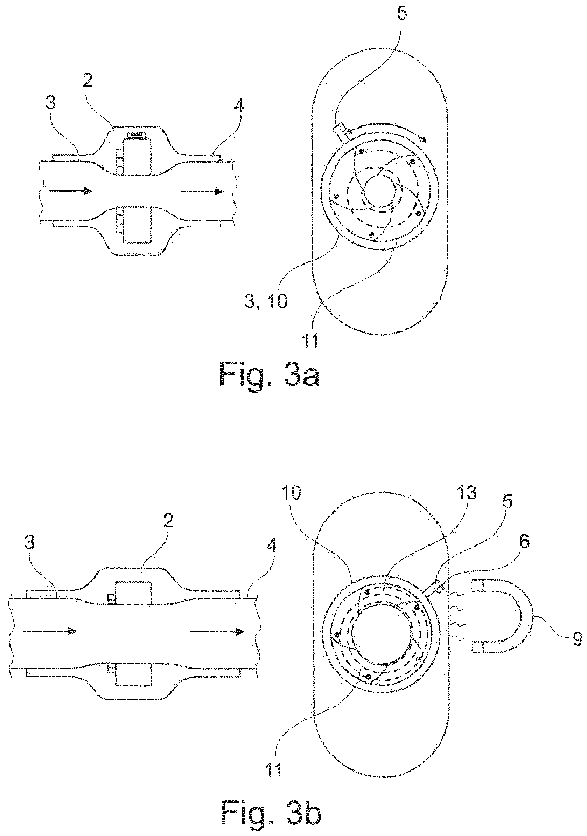

Figur 3 zeigt ein zum Einsatz in einem erfindungsgemäßen Implantat geeignetes Ventil, dessen Schließmechanik eine Irisblende 10 mit mehreren Lamellen 11 umfasst, die über eine Mechanik gemeinsam in den Durchlass geschwenkt werden können. In Figur 3a ist der verengte Normalzustand dargestellt, während in Figur 3b der erweiterte Zustand gezeigt wird, der während einer Dialysebehandlung eingenommen werden kann. Der Ventilkörper 2 umschließt dabei die Shunt-Leitung 4 und die Lamellen 11 verengen den Durchlass 3 je nach Zustand der Irisblende 10 in einem definierten Umfang und komprimieren dabei die kreisrunde und flexible Shunt-Leitung 4 gleichmäßig von allen Seiten, sodass die Kreisform der Shunt-Leitung 4 bei Verengung erhalten bleibt und sich der Mittelpunkt nicht verändert. Die Lamellen 11 sind am äußeren Umfang der Irisblende 10 mit einem Stößel 5 verbunden, wobei die Schließmechanik so ausgestaltet ist, dass eine Bewegung des Hebels entlang dem Umfang der Irisblende 10 zu einer Verengung oder Erweiterung des Durchlasses 3 führt. Der Stößel 5 trägt einen Permanentmagneten 6, sodass eine Bewegung des Stößels 5 durch Annäherung eines externen Magneten 9 ausgelöst werden kann. Die Lamellen 11 der Irisblende 10 sind mittels einer kreisförmigen Rückstellfeder 12 verbunden, sodass bei Entfernen des externen Magneten 9 der Stößel 5 und mithin die Lamellen 11 zur Ausgangsposition zurücckehren. Die Konfiguration der Irisblende 10 ist dergestalt, dass die Ausgangsposition (Figur 3a) einer Verengung des Durchlasses 3 und die aktivierte Position (Figur 3b) einer Erweiterung des Durchlasses 3 entspricht.

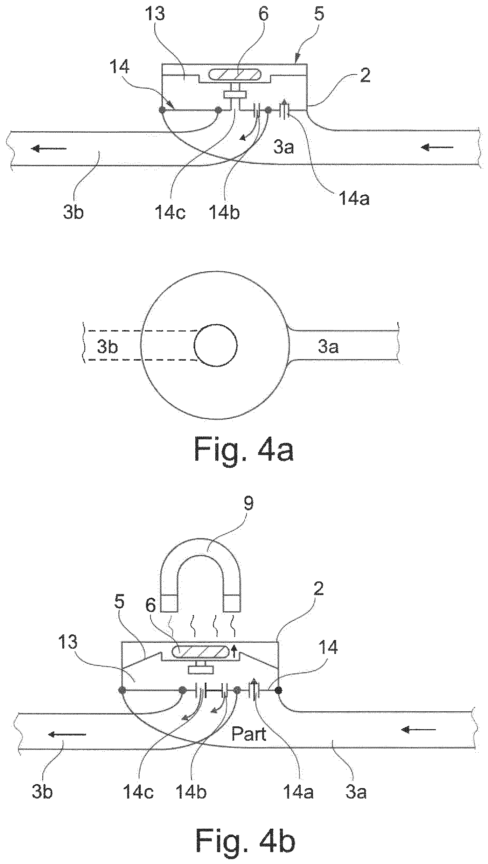

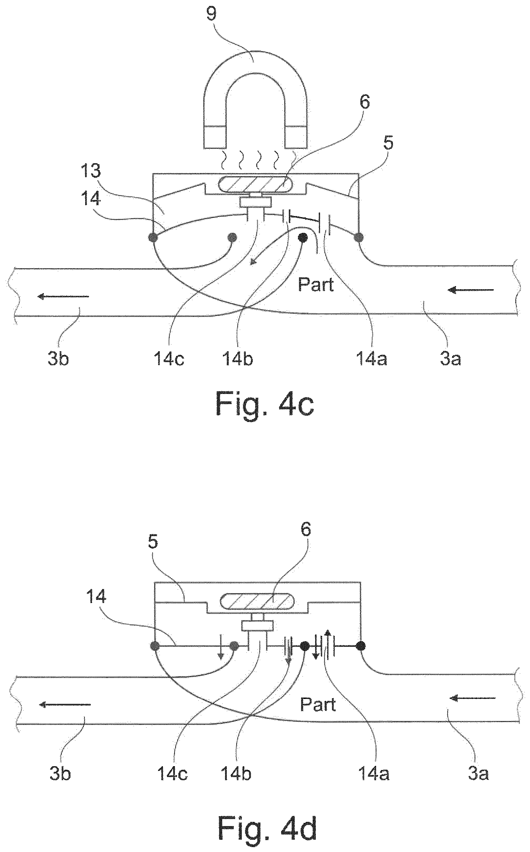

Figur 4 zeigt ein weiteres zum Einsatz in einem erfindungsgemäßen Implantat geeignetes Ventil, bei dem es sich um ein Servoventil handelt. Das Servoventil umfasst als zentrale Bestandteile einen Ventilkörper 2, in dem eine Ventilkammer 13 ausgebildet wird. Eine Durchlassmembran 14 trennt die Kammer 13 sowohl von einer den Vorlauf bildenden Leitung 3a des Durchlasses 3 als auch von einer den Rücklauf bildenden Leitung 3b des Durchlasses 3. Die den Rücklauf bildende Leitung 3b ist dabei im Sinne einer Rohr-in-Rohr-Anordnung in der den Vorlauf bildenden Leitung 3a aufgenommen, wobei die Leitungsabschnitte 3a und 3b durch ein Aufliegen der Durchlassmembran 14 auf der Öffnung des Innenrohres bzw. Leitungsabschnittes 3b getrennt sind. Die Durchlassmembran 14 weist drei Öffnungen auf, nämlich eine Vorlauföffnung 14a, welche die Ventilkammer 13 mit der den Vorlauf bildenden Leitung 3a verbindet, und zwei Rücklauföffnungen 14b und 14c, welche die Ventilkammer 13 mit der den Rücklauf bildenden Leitung 3a verbinden. Eine erste Rücklauföffnung 14b weist dabei einen Strömungsquerschnitt auf, der in etwa dem Strömungsquerschnitt der Vorlauföffnung entspricht. Der Strömungsquerschnitt der weiteren Rücklauföffnung 14c ist vorzugsweise größer.

Das Ventil umfasst einen Stößel 5, der so im Gehäuse 2 gelagert ist, dass er translatorisch darin verschoben werden kann. Der Stößel 5 ist fest mit einem Permanentmagneten 6 verbunden und weist an seiner Wirkfläche ein Schließelement auf, welches in Ruhestellung des Stößels 5 die vorzugsweise größere Rücklauföffnung 14c der Durchlassmembran 14 bedeckt und somit verschließt.

In Figur 4a ist der Normalbetrieb des Ventils dargestellt, in welcher der Stößel 5 sich in seiner durch Federspannung festgelegten Ausgangsposition befindet. Der Normalbetrieb entspricht dem Betrieb ohne Dialyse, bei dem nur ein geringer Blutfluss durch den Shunt erfolgen soll. Ein mit dem Pfeil symbolisierter Blutfluss durch den Vorlaufabschnitt 3a dringt über die Vorlauföffnung 14a in die Kammer 13 ein und verlässt diese durch die nicht überdeckte, kleinere Rücklauföffnung 14b wieder.

Wird der Stößel 5 durch einen externen Magneten 9 aus der Ruhestellung gehoben, wie in Figur 4b dargestellt, öffnet sich die größere Rücklauföffnung 14c der Durchlassmembran 14. Dies führt dazu, dass das Blut durch die nun beide geöffneten Rücklauföffnungen 14b und 14c schneller aus der Kammer 13 in den Rücklauf 3b abfließen kann, als es durch die Vorlauföffnung 14a nachfließen kann. Daraus resultiert ein Druckunterschied zwischen der Kammer 13 und der Vorlaufleitung 3a, durch welchen die Durchlassmembran 14 von der Öffnung des Leitungsabschnittes 3b gehoben wird, wie dies in Figur 4c dargestellt ist. In diesem Zustand kann eine große Menge an Blut direkt von der Vorlaufleitung 3a in die Rücklaufleitung 3b fließen, ohne die Kammer 13 durchlaufen zu müssen. Dies entspricht dem Zustand, der für eine Diaysebehandlung angestrebt wird.

Zur Rückführung des Ventils in die Ausgangsposition wird der externe Magnet 9 entfernt, wie in Figur 4d dargestellt. Der Stößel 5 federt dann in seine Ausgangsposition zurück, wodurch die größere Rücklauföffnung 14c wieder verschlossen wird und sich der Druck oberhalb und unterhalb der Membran 14 ausgleicht. Aufgrund der oben, also kammerseitig größeren Wirkfläche und der Eigenspannung der Membran 14 kehrt auch diese in die Ausgangsposition zurück und verschließt den Kurzschluss zwischen Vorlauf 3a und Rücklauf 3b wieder.

## Patentansprüche

1. Implantat zur Bereitstellung eines Shunts mit verstellbarem Durchfluss, wobei das Implantat ein Ventilelement mit einem Durchlass umfasst,
**dadurch gekennzeichnet,**
**dass** das Ventilelement einen zwischen einer Durchlassposition und einer Schließposition verfahrbaren Stößel umfasst, der einen Permanentmagneten aufweist und derart federbelastet ist, dass er in seine Schließposition vorgespannt ist.

2. Implantat nach Anspruch 1, **dadurch gekennzeichnet, dass** das Implantat eine durch den Durchlass verlaufende Leitung mit zumindest abschnittsweise flexiblen Wandungen umfasst.

3. Implantat nach einem der vorhergehenden Ansprüche, **dadurch gekennzeichnet, dass** der Stößel anhand einer Druckfeder wie beispielsweise einer Schraubendruckfeder, einer Tellerfeder oder einer Gummifeder federbelastet ist.

4. Implantat nach einem der vorhergehenden Ansprüche, **dadurch gekennzeichnet, dass** der Stößel in seiner Schließposition in den Durchlass ragt.

5. Implantat nach einem der Ansprüche 1 bis 3, **dadurch gekennzeichnet, dass** das Ventilelement ein Hydrauliksystem mit einem Angriffsbereich und einem am Durchlass angeordneten Wirkungsbereich aufweist, wobei der Stößel in seiner Schließposition derart Druck auf den Angriffsbereich ausübt, dass Hydraulikflüssigkeit verdrängt wird und am Wirkungsbereich des Hydrauliksystems eine Querschnittsverkleinerung des Durchlasses bewirkt.

6. Implantat nach Anspruch 5, **dadurch gekennzeichnet, dass** das Hydrauliksystem im Wirkungsbereich eine Ausstülpung aufweist, welche in den Durchlass ragt, wenn der Stößel in seiner Schließposition derart Druck auf den Angriffsbereich ausübt.

7. Implantat nach einem der Ansprüche 1 bis 3, **dadurch gekennzeichnet, dass** das Ventilelement eine am Durchlass angeordnete Schließmechanik aufweist und der Stößel derart mit dieser Schließmechanik in Verbindung steht, dass der Querschnitt des Durchlasses in der Schließposition des Stößels verkleinert wird, wobei vorzugsweise vorgesehen ist, dass die Schließmechanik eine Irisblende mit mehreren Lamellen umfasst, die über eine Mechanik gemeinsam in den Durchlass geschwenkt werden können.

8. Implantat nach einem der Ansprüche 1 bis 3, **dadurch gekennzeichnet, dass** es sich bei dem Ventilelement um ein Servoventil handelt, welches eine im Durchlass angeordnete Ventilkammer umfasst, die anhand einer Durchlassmembran sowohl von einem den Vorlauf bildenden Abschnitt des Durchlasses als auch von einem den Rücklauf bildenden Abschnitt des Durchlasses getrennt ist, wobei die Durchlassmembran lösbar auf dem den Rücklauf bildenden Durchlassabschnitt aufliegt und zwei Rücklauföffnungen aufweist, welche die Ventilkammer mit diesem Durchlassabschnitt verbinden, wobei eine der Rücklauföffnungen durch ein mit dem Stößel in Verbindung stehendes Schließelement lösbar verschlossen ist, und wobei die Durchlassmembran ferner eine Vorlauföffnung aufweist, welche die Ventilkammer mit dem den Vorlauf bildenden Durchlassabschnitt verbindet.

9. System umfassend ein Implantat nach einem der vorhergehenden Ansprüche sowie ein magnetisches Betätigungselement.

10. System nach Anspruch 9, **dadurch gekennzeichnet, dass** es sich bei dem magnetischen Betätigungselement um eine Armmanschette mit einem Gegenmagneten handelt.

## Claims

1. An implant for providing a shunt having an adjustable flow rate, wherein the implant comprises a valve element having a passage,
**characterized in that**
the valve element comprises a plunger that is travelable between a pass position and a closed position, that has a permanent magnet, and that is spring loaded such that it is preloaded into its closed position.

2. An implant in accordance with claim 1, **characterized in that** the implant comprises a line extending through the passage and having flexible walls at least sectionally.

3. An implant in accordance with one of the preceding claims, **characterized in that** the plunger is spring loaded by means of a compression spring such as a helical compression spring, a plate spring, or a rubber spring.

4. An implant in accordance with one of the preceding claims, **characterized in that** the plunger projects into the passage in its closed position.

5. An implant in accordance with one of the claims 1 to 3, **characterized in that** the valve element has a hydraulic system having a zone of attack and having an effective zone arranged at the passage, wherein the plunger exerts pressure on the zone of attack in its closed position such that hydraulic fluid is displaced and effects a reduction in the cross-section of the passage at the effective zone of the hydraulic system.

6. An implant in accordance with claim 5, **characterized in that** the hydraulic system has a protuberance in the effective zone that projects into the passage when the plunger exerts pressure on the zone of attack in its closed position in this manner.

7. An implant in accordance with one of the claims 1 to 3, **characterized in that** the valve element has a closing mechanism that is arranged at the passage; and **in that** the plunger is connected to this closing mechanism such that the cross-section of the passage is reduced in the closed position of the plunger, with provision preferably being made that the closing mechanism comprises an iris diaphragm having a plurality of blades that can be pivoted together into the passage via a mechanism.

8. An implant in accordance with one of the claims 1 to 3, **characterized in that** the valve element is a servo valve that comprises a valve chamber that is arranged in the passage and that is separated by a passage membrane both from a section of the passage forming the feed and from a section of the passage forming the return, with the passage membrane lying releasably on the passage section forming the return and having two return apertures that connect the valve chamber to this passage section, with one of the return apertures being releasably closed by a closing element connected to the plunger, and with the passage membrane furthermore having a feed aperture that connects the valve chamber to the passage section forming the feed.

9. A system comprising an implant in accordance with one of the preceding claims and a magnetic actuation element.

10. A system in accordance with claim 9, **characterized in that** the magnetic actuation element is an arm cuff having a counter-magnet.

## Revendications

1. Implant pour la réalisation d'un shunt à débit réglable, l'implant comprenant un élément de soupape pourvu d'un passage,
**caractérisé en ce que**
l'élément de soupape comprend un poussoir mobile entre une position de passage et une position de fermeture, qui présente un aimant permanent et qui est sollicité par ressort de manière à être précontraint dans sa position de fermeture.

2. Implant selon la revendication 1, **caractérisé en ce que** l'implant comprend un conduit s'étendant à travers le passage pourvu de parois flexibles au moins sur certaines parties.

3. Implant selon l'une des revendications précédentes, **caractérisé en ce que** le poussoir est sollicité par ressort à l'aide d'un ressort de compression, tel que par exemple un ressort de compression hélicoïdal, une rondelle-ressort ou un ressort en caoutchouc.

4. Implant selon l'une des revendications précédentes, **caractérisé en ce que**, dans sa position de fermeture, le poussoir dépasse dans le passage.

5. Implant selon l'une des revendications 1 à 3, **caractérisé en ce que** l'élément de soupape présente un système hydraulique pourvu d'une zone d'application et d'une zone d'action agencée sur le passage, le poussoir exerçant, dans sa position de fermeture, une telle pression sur la zone d'application que du liquide hydraulique est déplacé et provoque, sur la zone d'action du système hydraulique, un rétrécissement de section transversale du passage.

6. Implant selon la revendication 5, **caractérisé en ce que** le système hydraulique présente, dans la zone d'action, une excroissance qui dépasse dans le passage quand le poussoir, dans sa position de fermeture, exerce une telle pression sur la zone d'application.

7. Implant selon l'une des revendications 1 à 3, **caractérisé en ce que** l'élément de soupape présente un mécanisme de fermeture agencé sur le passage et le poussoir est relié à ce mécanisme de fermeture de telle manière que la section transversale du passage est réduite dans la position de fermeture du poussoir, le mécanisme de fermeture étant de préférence prévu de manière à comprendre un diaphragme à iris pourvu de plusieurs lamelles, qui peuvent être pivotées conjointement dans le passage par le biais d'un mécanisme.

8. Implant selon l'une des revendications 1 à 3, **caractérisé en ce que** l'élément de soupape est une servosoupape qui comprend une chambre de soupape agencée dans le passage, qui est séparée, à l'aide d'une membrane de passage, aussi bien d'une partie du passage formant l'arrivée que d'une partie du passage formant le retour, la membrane de passage reposant de manière séparable sur la partie de passage formant le retour et présentant deux ouvertures de retour qui relient la chambre de soupape à cette partie de passage, l'une des ouvertures de retour étant fermée de manière libérable par un élément de fermeture relié au poussoir et la membrane de passage présentant en outre une ouverture d'arrivée qui relie la chambre de soupape à la partie de passage formant l'arrivée.

9. Système comprenant un implant selon l'une des revendications précédentes ainsi qu'un élément d'actionnement magnétique.

10. Système selon la revendication 9, **caractérisé en ce que** l'élément d'actionnement magnétique est un brassard pourvu d'un contre-aimant.
